# EUROPEAN PATENT APPLICATION

(11) **EP 2 011 502 A1**
(43) Date of publication of application: **07.01.2009**
(21) Application number: 07111707.1
(22) Date of filing: 04.07.2007
(51) Int. Cl.: A61K 31/695, C07F 7/08, A61P 29/00

(54) **Silicon containing compounds and their use as anti-inflammatory agents**

(71) Applicant: Freie Universität Berlin, 14195 Berlin (DE)
(72) Inventor: Beckmann, Jens., 10713 Berlin (DE); Justies, Aileen., 13353 Berlin (DE)

(57) **Abstract**

The invention relates to a silicon containing compound of general formula I 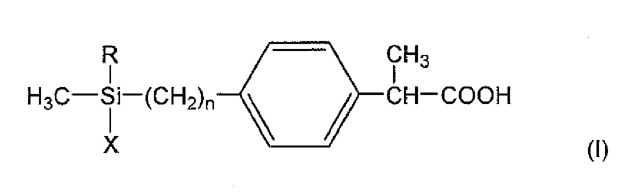 or their (R)- or (S)-enantiomer
or salts or esters thereof,
wherein
X represents hydrogen or deuterium
R represents CH₃, C₂H₅, CF₃ or C₂F₅
and
n is 1-3
and their use as anti-inflammatory agent.

## Description

The present invention relates to silicon containing compounds of general formula I, their (R)- or (S)-enantiomers or salts or esters thereof. The compounds are useful as analgesic, anti-inflammatory and antipyretic agents. Therefore, the invention is also directed to a pharmaceutical composition comprising as active substance one or more compounds of formula I and to the use of one or more compounds of formula I for the manufacture of a pharmaceutical composition for the prevention or treatment of an inflammatory disease, pain or fever. A process for preparing a silicon containing compound of formula I is also an object of the present invention.

More particularly, the invention concerns 2-(4-(dimethylsilylmethyl)phenyl) propionic acid and its amino acid salts, preferably the lysine salt.

It is well known in the art that ibuprofen or (+)-2-(4-isobutylphenyl) propionic acid (US 3,385,886), is a non-steroidal anti-inflammatory drug (NSAID) widely prescribed for its analgesic and anti-pyretic activity. It is also available as a low dose over-the-counter product to be used orally for the treatment of minor aches and pains. Besides it is topically applied as a gel for the treatment of muscular sprains and strains. Although ibuprofen is marketed as the racemic mixture, the (S)-enantiomer is known to be the active agent. In vivo testing, however, revealed the existence of an isomerase which converted (R)-ibuprofen to the active (S)-enantiomer. Thus, with some exceptions most ibuprofen formulations currently marketed are racemic mixtures.

In order to improve water solubility of ibuprofen, primarily to assist in the development of an injectable form, the lysine salt of ibuprofen has been developed (compare UK Patent Specification 1,471,910). Also high-content ibuprofen lysinate tablets (EP 0 505 180 A1), a pharmaceutical combination of ibuprofen lysine and domperidone (WO 00/21534) and a pharmaceutical combination of ibuprofen or their salts with aluminium hydroxide to mask the bitter taste of ibuprofen (US Patent 4,831,058) have been developed.

It was the object of the present invention to provide agents with anti-inflammatory activity comparable to that of ibuprofen and, preferably, improved pharmacological properties.

According to the invention a silicon containing compound of general formula I is provided 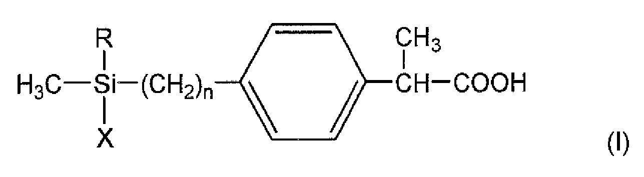 or their (R )- or (S)-enantiomer or salts or esters thereof, wherein
X represents hydrogen or deuterium
R represents CH₃, C₂H₅, CF₃ or C₂F₅
and n is 1-3.

In a preferred embodiment of the invention X in formula I represents hydrogen. In a further preferred embodiment R represents CH₃ or CF₃. n is preferably 1 or 2.

An especially preferred compound of formula I is 2-(4-(dimethylsilylmethyl)phenyl) propionic acid.

The above mentioned compounds of the invention include the pharmaceutically acceptable salts or esters, or any other compound which, upon administration to a human subject, is capable of providing directly or indirectly the therapeutically or preventively active metabolite.

Salts of compounds of formula I or their (R)- or (S)-enantiomers which may be conveniently used in prevention and therapy include physiologically acceptable base salts, e.g. derived from an appropriate base, such as alkali metal (e.g. sodium) salts, alkaline earth metal (e.g. magnesium) salts, ammonium salts or amino acid salts. According to the invention, especially preferred salts are the salts of basic amino acids, more particularly the lysine salt.

Esters of compounds of formula I or their (R)- or (S)-enantiomers include physiocologically acceptable esters with C₁-C₄-alcohols like methyl or ethyl alcohol, n-propyl or iso-propyl alcohol, n-butyl, iso-butyl, tert-butyl or sec-butyl alcohol. Esters with long chain alcohols like for instance PEG are also encompassed from the compounds of formula I.

A further object of the present invention is the process for preparing a silicon containing compound of general formula I 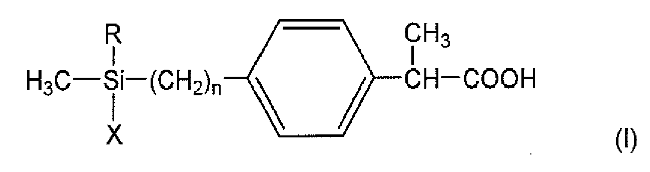 or their (R )- or (S)- enantiomer or salts or esters thereof, wherein
X represents hydrogen or deuterium
R represents CH₃, C₂H₅, CF₃ or C₂F₅
and n is 1-3
comprising reacting a compound of general formula II 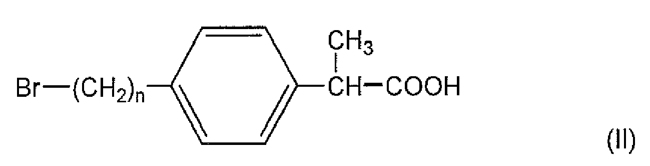 wherein n has the above meaning
with a compound of general formula III

XSi(CH₃)(R)Cl (III)

wherein X and R have the above meaning
in a solvent in the presence of a base and by treating with activated magnesium to obtain the acid of formula I, optionally separating the (R )- or (S)- enantiomer thereof or optionally reacting the obtained acid of formula I or the (R )- or (S)-enantiomer thereof with a base to obtain a salt thereof or optionally reacting the obtained acid of formula I or the (R )- or (S)- enantiomer thereof with an alcohol to obtain an ester thereof.

In a preferred embodiment of the invention triethylamine is used as the base. During the reaction the silyl chloride of general formula III also serves to protect the carbonic acid as silyl ester.

Thus, the compounds of the invention are synthesized in one step using commercially available chemicals. To obtain the (R)- or (S)-enantiomers the racemic compounds of formula I may be subjected to a resolution process as well known in the art or the enantiomers may be synthesized by asymmetric synthetis known in the art (e.g. using chiral auxiliary reagents and catalysts or transferring into diastereomers and separating them).

It has been found that the compounds of formula I especially 2-(4-(dimethylsilylmethyl)phenyl) propionic acid and its salts or esters, have improved pharmacological properties in comparison to ibuprofen and their derivates. The silicon containing compounds of the present invention show improved body absorption and length of stay in the body (longer elimination half lifes). Hence, a smaller daily dose is required.

Therefore, the present invention is also directed to a pharmaceutical composition comprising as active substance one or more silicon containing compounds of formula I in an amount sufficient to exhibit a therapeutic or preventive effect.

The pharmaceutical compositions may also comprise conventional auxiliary substances, preferably carriers, adjuvants and/or vehicles. For example, said carriers can be fillers, extenders, binders, humectants, disintegrants, dissolution retarders, absorption enhancers, wetting agents, adsorbents, and/or lubricants.

The pharmaceutical compositions of the invention may be prepared as a gel, powder, tablet, sustained-release tablet, premix, emulsion, infusion formulation, drops, concentrate, granulate, syrup, pellet, bolus, capsule, aerosol, spray or inhalant and/or used in this form.

For example, the pharmaceutical composition of the present invention can be administered orally in any orally tolerable dosage form, including capsules, tablets and aqueous suspensions and solutions, without being restricted thereto. In case of tablets for oral application, carriers frequently used include microcrystalline cellulose, lactose and corn starch. Typically, lubricants such as magnesium stearate can be added. For oral administration in the form of capsules, useful diluents such as lactose and dried corn starch are employed. In oral administration of aqueous suspensions the active substance is combined with emulsifiers and suspending agents. Also, particular sweeteners and/or flavors and/or coloring agents can be added, if desired.

The compounds of formula I can also be present in micro-encapsulated form, optionally with one or more of the above-specified carriers.

In addition to the compounds of formula I as active substance(s), suppositories may include conventional water-soluble or water-insoluble carriers such as polyethylene glycols, fats, e.g. cocoa fat and higher esters (for example, C₁₄ alcohols with C₁₆ fatty acids) or mixtures of these substances.

In addition to the compounds of formula I as active substance(s), ointments, pastes, creams and gels may include conventional carriers such as animal and vegetable fats, waxes, paraffins, starch, tragacanth, cellulose derivatives, polyethylene glycols, silicones, bentonites, silicic acid, talc and zinc oxide or mixtures of these substances.

If the pharmaceutical composition according to the invention is provided as solution or emulsion it may include conventional carriers such as solvents, solubilizers, and emulsifiers such as water, ethyl alcohol, isopropyl alcohol, ethyl carbonate, ethyl acetate, benzyl alcohol, benzyl benzoate, propylene glycol, 1,3-butylene glycol, dimethylformamide, oils, especially cotton seed oil, peanut oil, corn oil, olive oil, castor oil and sesame oil, glycerol, glycerol formal, tetrahydrofurfuryl alcohol, polyethylene glycols, and fatty esters of sorbitan, or mixtures of these substances. For parenteral application, the solutions and emulsions may also be present in a sterile and blood-isotonic form.

In addition to the compounds of formula I as active substance(s), suspensions may include conventional carriers such as liquid diluents, e.g. water, ethyl alcohol, propylene glycol, suspending agents, e.g. ethoxylated isostearyl alcohols, polyoxyethylene-sorbitol and sorbitan esters, microcrystalline cellulose, aluminum metahydroxide, bentonite, agar, and tragacanth, or mixtures of these substances.

The pharmaceutical compositions can be present in the form of a lyophilized sterile injectable formulation, e.g. as a sterile injectable aqueous solution or aqueous or oily suspension. Such a suspension can also be formulated by means of methods known in the art, using suitable dispersing or wetting agents (such as Tween 80) and suspending agents.

The production of the pharmaceutical formulations specified above proceeds in a usual manner according to well-known methods, e.g. by mixing the active substance(s) with the carrier(s).

According to the invention the compounds of formula I are incorporated in a pharmaceutical formulation at a concentration of 0.1 to 99.5, preferably 0.5 to 95, and more preferably 20 to 80 wt.-%. That is, the active substance is present in the above pharmaceutical formulations, e.g. tablets, pills, granulates and others, at a concentration of preferably 0.1 to 99.5 wt.-% of the overall mixture.

In a preferred embodiment of the invention the oral dose of the compounds of formula I is 100-400 mg, preferably 100-200 mg, every 4 to 6 hours. However, the dose varies with body mass and indication. The recommended maximum daily dose is about 1.200 mg.

According to a further embodiment, the present invention concerns the use of one or more compounds according to formula I for the manufacture of a pharmaceutical composition for the prevention or treatment of an inflammatory disease, pain or fever.

Inflammatory diseases which can be treated or prevented by the compounds of formula I include, but are not limited to arthritis, rheumatism, gout, Crohn's disease, ulcerative colitis or primary dysmenorrhoea. In a further aspect of the invention the compounds of formula I are suitable in prevention and therapy of pain and fever. They are particularly well suited for treatment of headache, migraine and any pain associated with an inflammatory component.

According to a further embodiment, the present invention provides a method for treating an inflammatory disease, pain and/or fever comprising administering to a patient suffering from that disorder a therapeutically and safe amount of at least one compound of formula I. "Therapeutically effective amount" means an amount effective to yield the desired therapeutic response. "Safe amount" refers to the quantity of a component that does not cause undue adverse side effects (such as toxicity, irritation, or allergic response) commensurate with a reasonable benefit/risk ratio when used in the manner of this invention.

Without intending to be limiting, the invention will be explained in more detail with reference to the following examples.

**Synthesis of 2-(4-(dimethylsilylmethyl)phenyl) propionic acid. A 100 mL** Schlenk tube was charged with 2-(4-(bromomethyl)phenyl) propionic acid (5.00 g, 20.6 mmol), triethylamine (4.16g, 41.1 mmol), dimethylchlorosilane (7.79 g, 82.3 mmol) and diethylether (50 mL). The mixture stirred for 12 h before the colorless precipitate was removed by filtration and washed with diethyl ether (50 mL). The combined ether layers were added to activated Mg turnings and heated under reflux for 12 h. The mixture was hydrolyzed by addition of ice water (100 mL). The layers were separated and the organic layer was dried over Na₂SO₄. The solvent was removed under reduced pressure and the crude oily product was purified by column chromatography using silica gel and hexane I ethyl acetate = 4:1. Yield 85%, mp. 45-47.5 °C 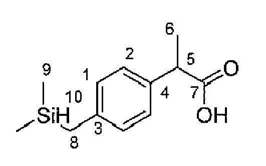 **¹H-NMR** (Aceton-d₆) δ = 7.20 (d, *³J= 8.1* Hz, 2H, H-1,), 7.03 (d, *³J=* 8.1 Hz, 2H H-2,), 3.91-3.95 (m(sept), *³J=3.5 Hz,* 1 H, H-10), 3.68 (quart, *³J=* 7.2 *Hz,* 1 H, H-5), 2.16 (d, *³J=* 3.2 *Hz,* 2 H, H-8), 1.40 (d, *³J= 7.2 Hz,* 3 H, H-6), 0.05 (d, *³J=* 3.7 Hz, 6 H, H-9) ppm. ¹³**C-{1H}-NMR** (Aceton-d₆) δ = 175.8 (C-7), 139.4 (C_{quart}), 138.0(C_{quart}), 129.0(Cₐᵣₒₘ,C-1), 128.2(Cₐᵣₒₘ, C-2), 45.2 (C-5), 23.9 (C-8), 19.1 (C-6), -4.7 (C-9) ppm. ²⁹**Si-{1H)-NMR** (Aceton-d₆)δ = 11.5 ppm. **Microanalysis** Calc. for C₁₂H₁₈O₂Si (222.36) C, 64.82; H, 8.16; Found C, 64.54; H, 8.55. **MS** m/z = 222.4 (54%, [M]⁺), 204.9 (15%, [M-H₂O]⁺), 191.9 (3%, [M-CO₂H₂]⁺), 178.8 (2%, [M-C₂H₅O₂]⁺), 163.9 (37%, [M-SiC₂H₇]⁺ or [M-C₂H₂O₂]⁺), 147.9 (3%, M-SiC₂H₉O]⁺) , 119.9 (11 %, [M-SiC₃H₆O₂]⁺), 119.0 (100%, [C₉H₁₁]⁺).

## Claims

1. A silicon containing compound of general formula I 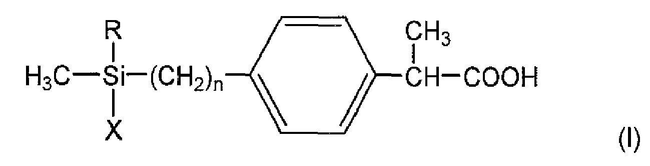 or their (R )- or (S)-enantiomer
or salts or esters thereof,
wherein
X represents hydrogen or deuterium
R represents CH₃, C₂H₅, CF₃ or C₂F₅
and
n is 1-3.

2. The compound according to claim 1, wherein X represents hydrogen.

3. The compound according to claim 1 or 2, wherein R represents CH₃ or CF₃.

4. The compound according to anyone of claims 1 to 3, wherein n is 1 or 2.

5. The compound according to anyone of claims 1 to 4 being 2-(4-(dimethylsilylmethyl)phenyl) propionic acid.

6. The compound according to anyone of claims 1 to 5 being a salt of a basic amino acid, preferably the lysine salt.

7. A process for preparing a silicon containing compound of general formula 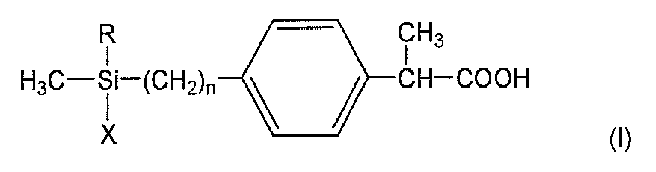 or their (R )- or (S)- enantiomer
or salts or esters thereof,
wherein
X represents hydrogen or deuterium
R represents CH₃, C₂H₅, CF₃ or C₂F₅
and
n is 1-3
comprising reacting a compound of general formula II 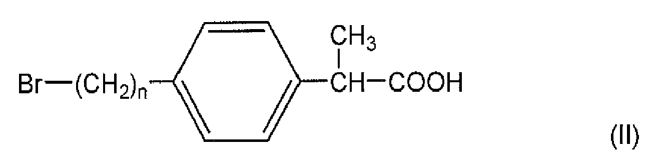 wherein n has the above meaning
with a compound of general formula III
XSi(CH₃)(R)Cl (III)
wherein X and R have the above meaning
in a solvent in the presence of a base and by treating with activated magnesium to obtain the acid of formula I, optionally separating the (R)- or (S)-enantiomer thereof or optionally reacting the obtained acid of formula I or the (R)- or (S)-enantiomer thereof with a base to obtain a salt thereof or optionally reacting the obtained acid of formula I or the (R)- or (S)-enantiomer thereof with an alcohol to obtain an ester thereof.

8. The process according to claim 7, wherein the used base is triethylamine.

9. The process according to claim 7 or 8, wherein the obtained acid is reacted with a basic amino acid to obtain an amino acid salt.

10. A pharmaceutical composition comprising as active substance one or more silicon containing compounds according to claims 1 to 6 in an amount sufficient to exhibit a therapeutic or preventive effect.

11. Use of one or more compounds according to claims 1 to 6 for the manufacture of a pharmaceutical composition for the prevention or treatment of an inflammatory disease, pain or fever.

12. Use according to claim 11, wherein the inflammatory disease is selected from the group comprising arthritis, rheumatism, gout, Crohn's disease, ulcerative colitis or primary dysmenorrhoea.
